# EUROPEAN PATENT APPLICATION

(11) **EP 0 578 112 A2**
(43) Date of publication of application: **12.01.1994**
(21) Application number: 93110409.5
(22) Date of filing: 30.06.1993
(51) Int. Cl.: C07H 19/04, C07H 15/10, C07H 15/14

(54) **Disaccharide derivatives and processes for the preparation thereof**

(30) Priority: 03.07.1992 JP 176539/92
(71) Applicant: The Nisshin Oil Mills, Ltd., Tokyo (JP)
(72) Inventor: Yamada, Yutaka, The Nisshin Oil Mills,Ltd., Yokohama-shi, Kanagawa-ken (JP); Kameyama, Akihiko, The Nisshin Oil Mills,Ltd., Yokohama-shi, Kanagawa-ken (JP)
(74) Representative: Türk, Gille, Hrabal, Leifert

(57) **Abstract**

Disaccharide derivatives selected from the group consisting of the compounds represented by formulas (I) and (II) 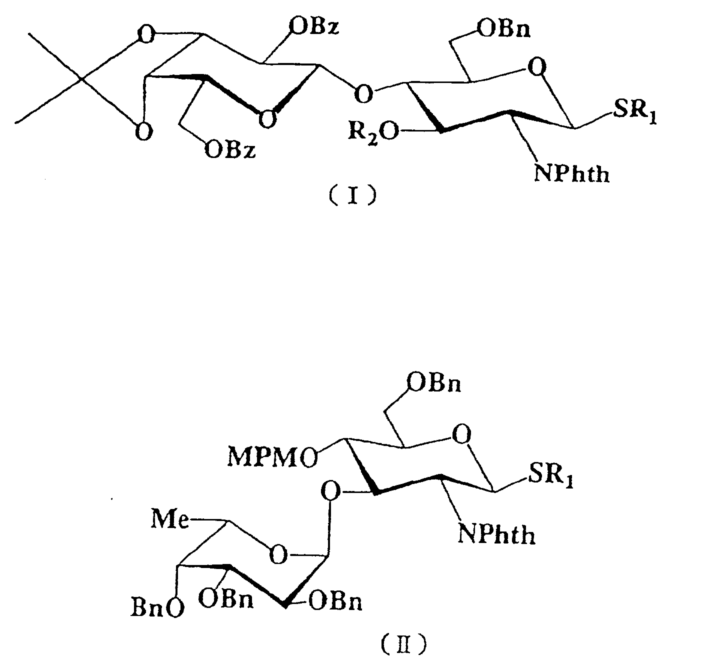
in which R₁ represents C₁-C₄ alkyl or phenyl, R₂ represents benzyl or methoxybenzyl, Bn stands for benzyl, Bz stands for benzoyl, MPM stands for methoxybensyl, Me stands for methyl and NPhth stands for phthalimide of the formula 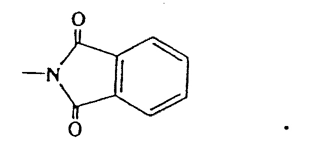
Those derivatives can be used as intermediates for the preparation of sialyl SSEA-1 ganglioside and other lacto-and neolacto-series gangliosides which are useful as tumor markers in a serum diagnosis of cancers.

## Description

### FIELD OF THE INVENTION

This invention relates to new disaccharide derivatives useful as intermediates in the synthesis of various glycolipids, processes for the preparation thereof and the use of those derivatives for the manufacture of gangliosides, especially sialyl SSEA-1 (stage-specific embryonic antigen-1).

### BACKGROUND OF THE INVENTION

Sphingoglycolipids present in cells are to be expected for the elucidation of the biochemical mechanism and for the therapeutic application, since they are dramatically transformed with the cycle, proliferation, differentiation and canceration of cells. However, a large number of glycolipids including sialyl SSEA-1 suggested to be involved in the metastatic formation of cancer cells have not been synthesized or have been difficult to synthesize.

In the preparation of monoclonal antibodies against sialyl SSEA-1, intact cancer cells or glycolipids purified from cancer cells have been used as an immunogen (Kannagi et al., Byori to Rinsho (Pathology and Clinic), Vol. 8, 6. "Glycolipids, Saccharide Chain Antigen", pp. 85-99, Bunkodo Publisher). If intact cancer cells are used as an immunogen, there is little likelihood of preparing a desired antibody, because of its being contaminated with a variety of antigens. If purified glycolipids are used, it is often difficult to purify glycolipids in an amount enough to prepare monoclonal antibodies. Even purified glycolipids are not considered to be a completely single molecule, since a ceramide moiety consists of a mixture of compounds with different numbers of unsaturated bond, different lengths of hydrocarbon chain or the like. To solve such problems, many attempts have been made to chemically synthesize interesting glycolipids for the use as an immunogen (Ogawa T, Kannagi R et al., The Journal of Biological Chemistry, Vol. 262, No. 3, pp. 1358-1362, 1987). However, no successful results have been achieved. So far as sialyl SSEA-1 is concerned, there is no report on its synthesis. One of the reasons why such glycolipids are difficult to chemically synthesize is the variety of reactive groups in the starting saccharides and the diversity of the reaction products. Thus, it has been required that intermediate compounds are clarified for the synthesis of polysaccharides having the desired specific structure and a general process of preparing polysaccharides is accomplished using such intermediate compounds.

### SUMMARY OF THE INVENTION

Thus, an object of the invention is to provide new disaccharide derivatives useful as intermediates in the synthesis of glycolipids, as well as processes of producing such derivatives.

The present invention is concerned with disaccharide derivatives selected from the group consisting of the compounds represented by formulas (I) and (II) 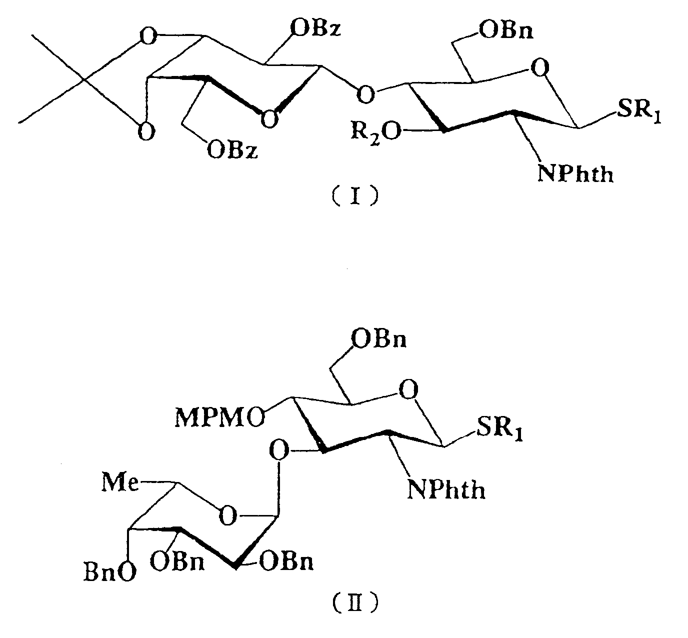
in which R₁ represents C₁-C₄ alkyl or phenyl, R₂ represents benzyl or methoxybenzyl, Bn stands for benzyl, Bz stands for benzoyl, MPM stands for methoxybenzyl, Me stands for methyl and NPhth stands for phthalimide of the formula

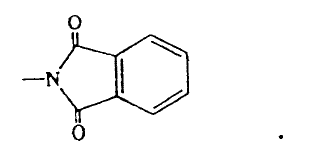

### DETAILED DESCRIPTION OF THE INVENTION

The disaccharide derivatives of the invention are characterized in that functional groups such as a hydroxyl group or the like present in the molecule are suitably protected or substituted so as to enable the architecture of a repeating structure (a variety of saccharide chain structure) of a galactosylglucosamine (lactosamine) which is a characteristic partial structure in lacto- and neolacto-series glycolipids
[(Galβ1→3/4GlcNAcβ1→3)nGalβ1→4Glcβ1→Cer, n≧1].

Those disaccharide derivatives can be produced by more simple and economical way that to prepare similar prior compounds.

The preparation of such disaccharide derivatives will be explained below by reference to the following reaction schemes.

The abbreviations used in the explanation of the reaction schemes and the following description have the following meanings.
Ac, acetyl; Me, methyl; Et, ethyl; Pr, propyl; i-Pr, isopropyl; Bu, butyl; t-Bu, t-butyl; Ph, phenyl; Bn, benzyl: MPM, p-methoxybenzyl; NPhth, phthalimido; Bz, benzoyl; SE, 2-trimethylsilylethyl; Tf, trifluoromethanesulfonyl; THF, tetrahydrofuran; DMF, dimethylformamide; MS, molecular sieves; DMTST, dimethyl methylthiosulfonium trifluoromethanesulfonate; DDQ, 2,3-dichloro-5,6-dicyano-p-benzoquinone; TFA, trifluoroacetic acid; DBU, 1,8-diazabicyclo(5.4.0)undec-7-ene; WSC, 1-ethyl-3-(dimethylaminopropyl)carbodiimide hydrochloride.

First, the preparation of methyl O-(2,6-di-O-benzoyl-3,4-O-isopropylidene-β-D-galactopyranosyl)-(1→4)-3,6-di-O-benzyl-2-deoxy-2-phthalimido-1-thio-β-D-glocopyranoside (Compound 6) which is one of the compounds of formula (I) will be illustrated by Reaction Scheme 1, starting from methyl 2,6-di-O-benzoyl-3,4-O-isopropylidene-1-thio-β-D-galactopyranoside (Compound 1) and methyl 4,6-O-benzylidene-2-deoxy-2-phthalimido-1-thio-β-D-glucopyranoside (Compound 3). 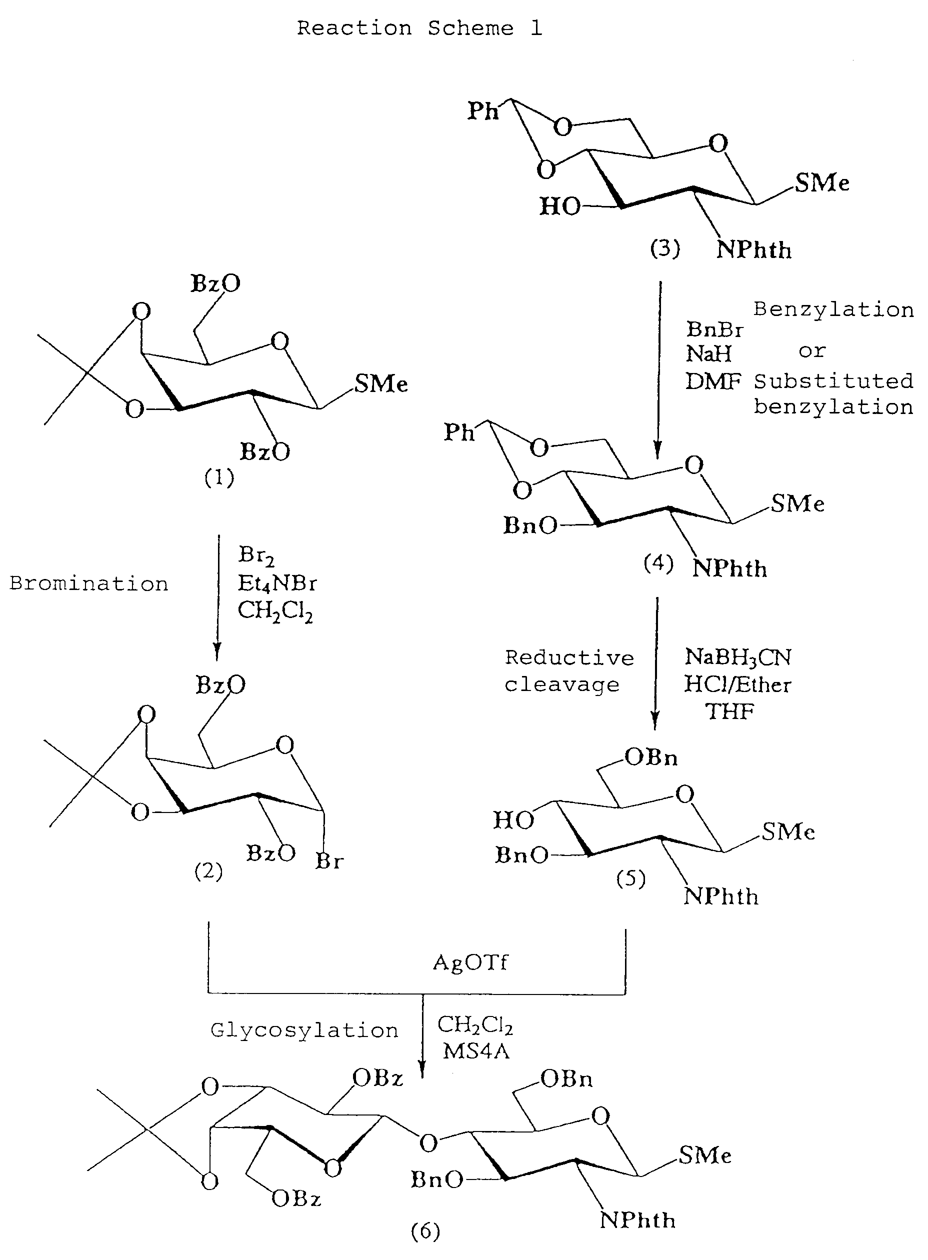
The starting Compounds (1) and (3) can be prepared according to known methods, e.g., by the methods mentioned in Per J. Garegg et al., Carbohydrate Research, 136 (1985) 207-213 and Ogawa T et al., Carbohydrate Research, 155 (1986) C1-C5, which are incorporated herein by reference.

In the first step, Compound (1) is treated with an equivalent amount of bromine and a catalytic amount of Et₄NBr in CH₂Cl₂ at -20°C to afford Compound (2), 2,6-di-O-benzoyl-3,4-O-isopropylidene-α-D-galactopyranosylbromide, as a sugar donor. Compound (2) is purified and immediately used for subsequent condensation reaction (glycosylation), because of its relatively unstable material.

In the second step, Compound (3) is reacted at the 3-hydroxyl group with sodium hydride, e.g., in DMF to form the corresponding alkoxide, which is then reacted with BnBr at room temperature to afford Compound (4), 3-O-benzyl-4,6-O-benzylidene-2-deoxy-2-phthalimido-1-thio-β-D-glucopyranoside. Then, the benzylidene group incorporated between 4- and 6-positions of Compound (4) is subjected to reductive cleavage with a large excess of NaBH₃CN and HCl/ether, e.g., in THF to give Compound (5), methyl-3,6-di-O-benzyl-2-deoxy-2-phthalimido-1-thio-β-D-glucopyranoside, as a sugar acceptor. The sugar donor (2) and the sugar acceptor (5) are subjected to condensation reaction (glycosylation) to afford Compound (6). The condensation reaction is conducted at a temperature of -80°C to 0°C for 10 to 30 hrs., in the presence of AgOTf as a promoter in CH₂Cl₂ thoroughly dehydrated by molecular sieves (MS)-4A.

The purification of the reaction products formed in the process steps and the objective compound is performed by a column chromatography on silica gel conventionally employed in the art. Unless otherwise stated, all the compounds in the present invention are purified by this method.

In addition to Compound (6) as prepared above, other compounds of formula (I) include the following Compounds (20) to (32) with R₁ and R₂ being as listed below.

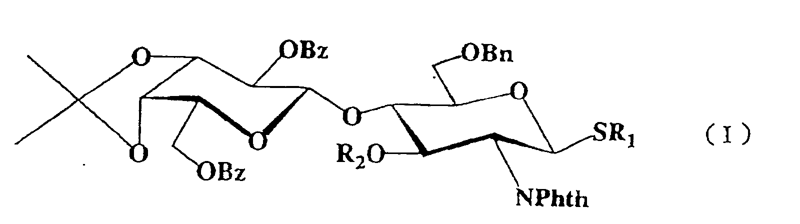

| | R₁ | R₂ |
|---|---|---|
| (20) | Et | Bn |
| (21) | Pr | Bn |
| (22) | i-Pr | Bn |
| (23) | Bu | Bn |
| (24) | t-Bu | Bn |
| (25) | Ph | Bn |
| (26) | Me | MPM |
| (27) | Et | MPM |
| (28) | Pr | MPM |
| (29) | i-Pr | MPM |
| (30) | Bu | MPM |
| (31) | t-Bu | MPM |
| (32) | Ph | MPM |

Compounds (20) to (32) can be prepared starting from the corresponding thioglycosides, e.g., ethyl-, propyl-, isopropyl-, butyl-, t-butyl- and phenyl-thioglycosides, in a similar manner as shown in Reaction Scheme 1 above. That is, the starting thioglycoside compounds are respectively converted into the compounds of the following formula (III), sugar acceptors (7) to (12) using BnBr or into sugar acceptors (13) to (19) using MPMCl.

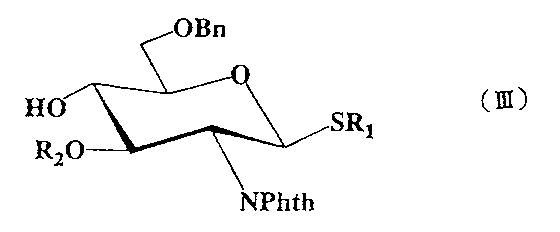

| | R¹ | R₂ |
|---|---|---|
| (7) | Et | Bn |
| (8) | Pr | Bn |
| (9) | i-Pr | Bn |
| (10) | Bu | Bn |
| (11) | t-Bu | Bn |
| (12) | Ph | Bn |
| (13) | Me | MPM |
| (14) | Et | MPM |
| (15) | Pr | MPM |
| (16) | i-Pr | MPM |
| (17) | Bu | MPM |
| (18) | t-Bu | MPM |
| (19) | Ph | MPM |

Subsequently, those sugar acceptors (7) to (19) are reacted with the sugar donor (2) to provide the objective compounds (20) to (32).

Secondly, the preparation of methyl O-(2,3,4-tri-O-benzyl-α-L-fucopyranosyl)-(1→3)-6-O-benzyl-2-deoxy-4-O-methoxybenzyl-2-phthalimido-1-thio-β-D-glucopyranoside (Compound 36) which is one of the compounds of formula (II) will be illustrated by Reaction Scheme 2, starting from methyl 4,6-O-benzylidene-2-deoxy-2-phthalimido-1-thio-β-D-glucopyranoside (Compound 3) and methyl 2,3,4-tri-O-benzyl-1-thio-β-L-fucopyranoside (Compound 33). 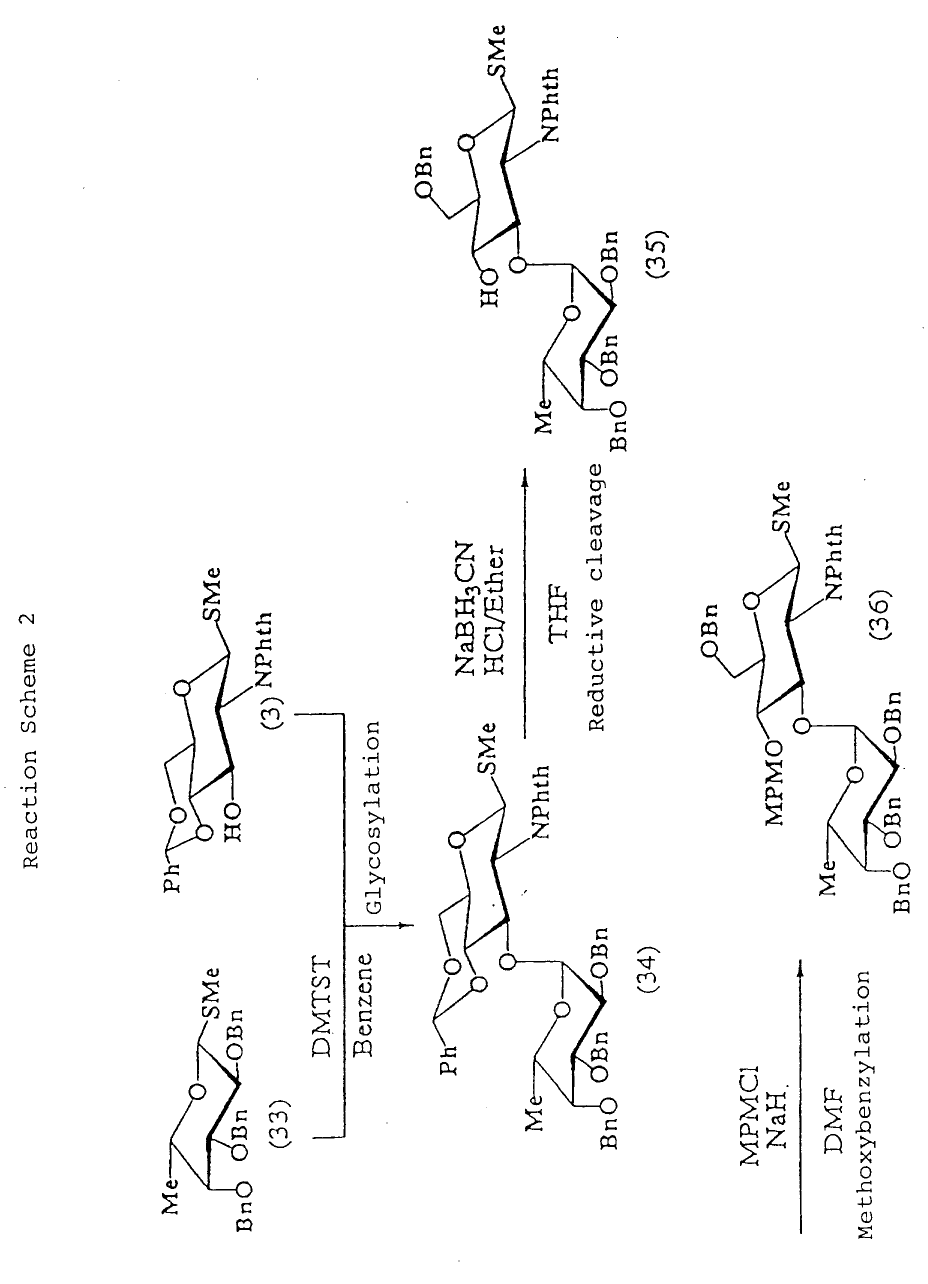
In the first step, known Compound (33) is condensed with Compound (3) using DMTST as a promoter for condensation, in benzene at a temperature of -50°C to room temperature to afford methyl O-(2,3,4-tri-O-benzyl-α-L-fucopyranosyl)-(1→3)-4,6-O-benzylidene-deoxy-2-phthalimido-1-thio-β-D-glucopyranoside (Compound 34).

Then, Compound (34) is converted by reductive cleavage of the benzylidene group into methyl O-(2,3,4-tri-O-benzyl-α-L-fucopyranosyl)-(1→3)-6-O-benzyl-2-deoxy-2-phthalimido-1-thio-β-D-glucopyranoside (Compound 35), followed by introducing methoxybenzyl to free hydroxyl of Compound (6) in a similar manner as mentioned above, thus providing the objective compound of the invention, Compound (36).

In addition to Compound (36) as prepared above, other compounds of formula (II) include the following Compounds (37) to (42) with R₁ being as listed below.

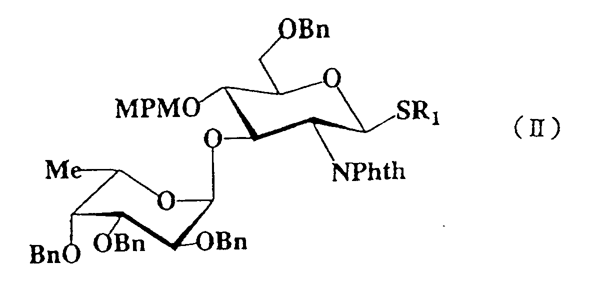

| | |
|---|---|
| (37) | Et |
| (38) | Pr |
| (39) | i-Pr |
| (40) | Bu |
| (41) | t-Bu |
| (42) | Ph |

Compounds (37) to (42) can be prepared starting from the corresponding thioglycosides, e.g., ethyl-, propyl-, isopropyl-, butyl-, t-butyl- and phenyl-thioglycosides, in a similar manner as shown in Reaction Scheme 2 above.

The disaccharide derivatives of formulas (I) and (II) are useful intermediates for the synthesis of sialyl SSEA-1 ganglioside and other lacto- and neolacto-series gangliosides having the fundamental structure of (Galβ1→ 3/4GlcNAcβ1→3)nGalβ1→4Glcβ1→Cer, n≧1, which are useful as tumor markers in a serum diagnosis of cancers. The compounds of formula (I) are glycosyl donors and have necessary function to construct the structure of Galβ1→ 4GlcNAcβ1→, that is, hydroxyl groups at 3- and 4-positions of galactose being selectively deprotected and the anomeric position of glucosamine being substituted by a sugar donor group. The compounds of formula (II) are glycosyl acceptors and have necessary function to form the structure of 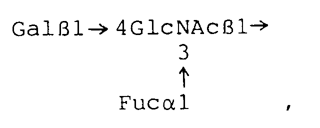
that is, the hydroxyl group at 4-position of glucosamine being selectively deprotected. Thus, using the compounds of formulas (I) and (II) enables the systematized synthesis of glycolipids, representative of which is sialyl SSEA-1. In addition to the synthesis of sialyl SSEA-1, for instance, the compound which is prepared by deprotecting the methoxybenzyl group of the compound (II) followed by condensation with Compound (2) can be introduced into the saccharide chain, thus constructing extended saccharide chain having fucose incorporated into the middle of the chain.

Using, for instance, Compounds (6) and (36) of the present invention, the present inventors were successful in the preparation of sialyl SSEA-1 (VI³NeuAcV³ FucnLc₆-Cer) which could not been synthesized hitherto.

The use of the present compounds as intermediates in the synthesis of polysaccharides is illustrated below, taking the synthesis of sialyl SSEA-1 as an example. The synthetic route starting from Compound (6) and leading to sialyl SSEA-1 is shown by the following Reaction Schemes 3, 4 and 5. 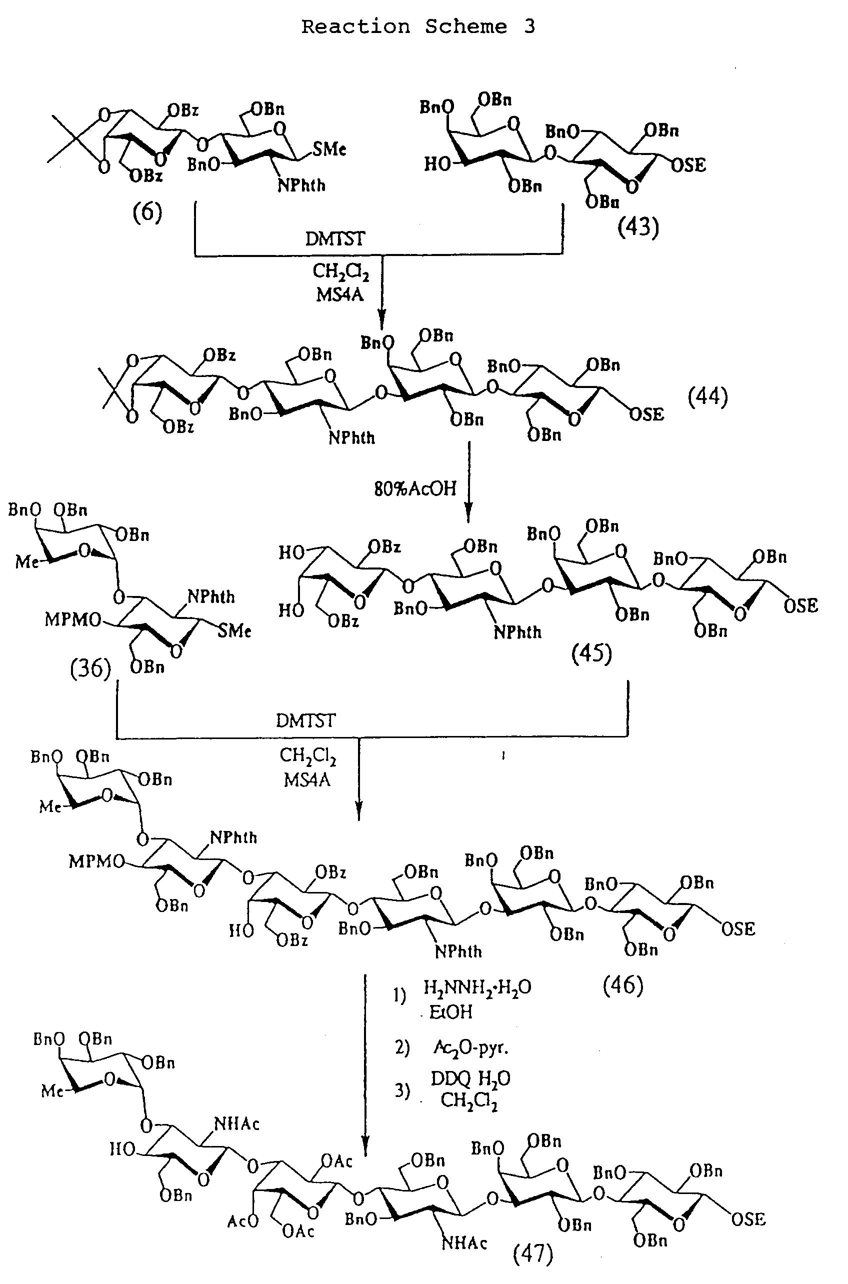 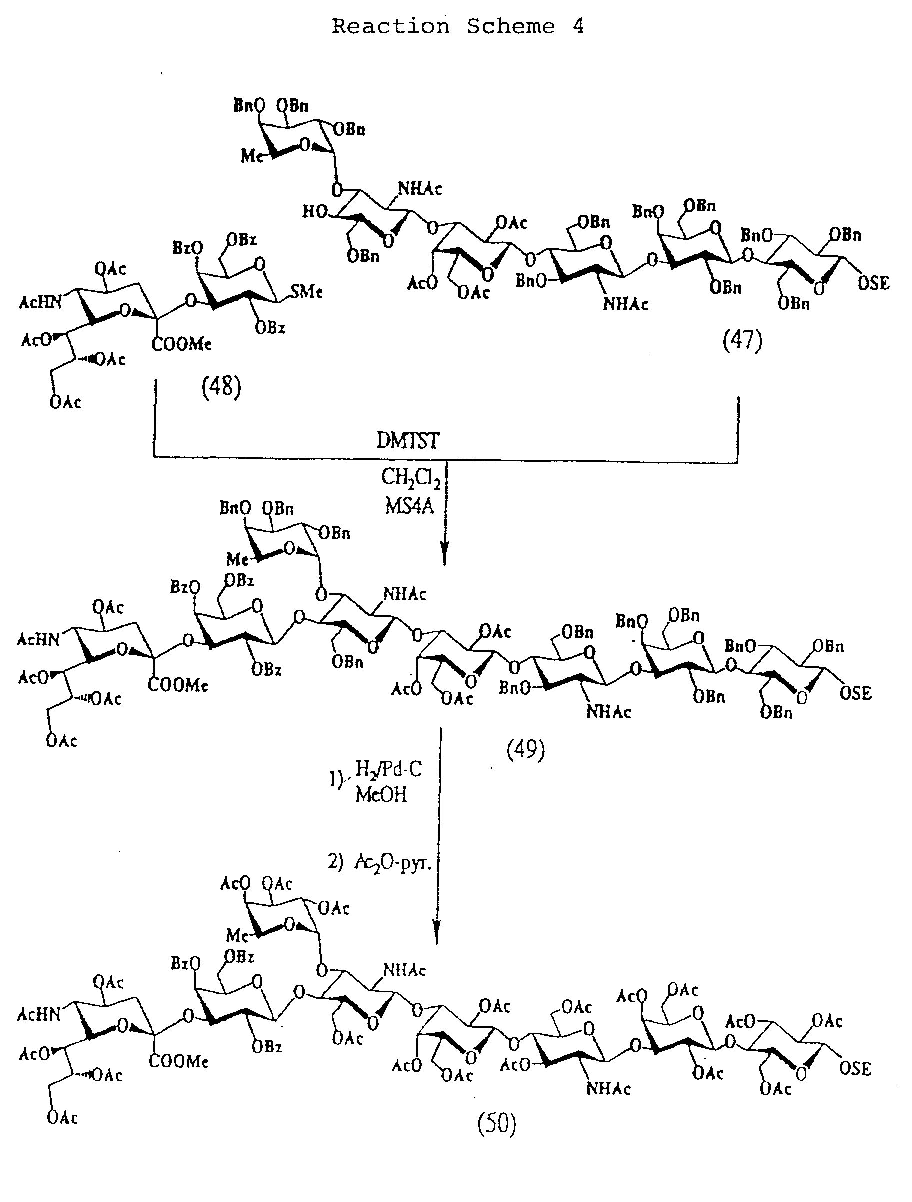 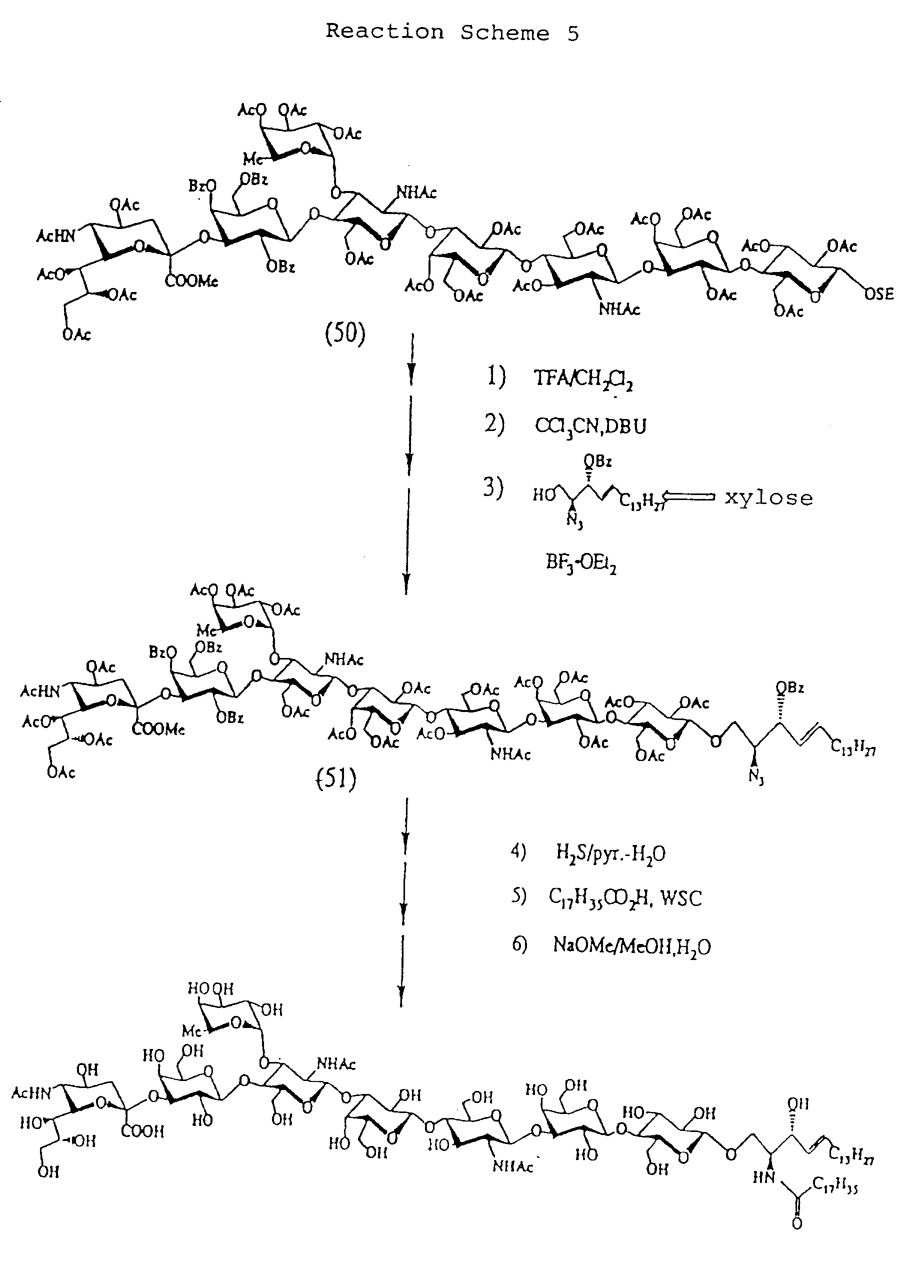
In the first step, Compound (6) of the present invention and known Compound (43) are subjected to condensation reaction in the presence of DMTST as a promoter to give Compound (44). Removal of a reducing terminus, isopropylidene group in Compound (44) under an acidic condition affords Compound (45). This compound as a sugar acceptor is condensed with Compound (36) of the present invention as a sugar donor in the presence of DMTST as a promoter to give a regio-selective condensation product (46). Two phthalimido groups in Compound (46) are converted by hydrazine monohydrate into amino groups. Acetylation of those amino groups and free hydroxyl group with acetic anhydride/pyridine and oxidative removal of the methoxybenzyl group with DDQ gives Compound (47). DMTST-promoted condensation of Compound (47) with a sugar donor, sialylgalactose derivative (48) affords octasaccharide Compound (49). The sialylgalactose derivative (48) is known compound by Kameyama A et al., Journal of Carbohydrate Chemistry, 10 (4), 549-560, 1991. Reductive removal of 12 benzyl groups in Compound (49) with hydrogen in the presence of a Pd-C catalyst followed by acetylation of free hydroxyl group with acetic anhydride/pyridine provides Compound (50) wherein all the hydroxyl groups except for 2-(trimethylsilyl)ethyl (SE) group of a non-reducing terminus were protected by an acyl type protecting group.

The process of introducing a ceramide moiety into the saccharide chain is shown by Reaction Scheme 5. This process is the same as that reported by Kameyama et al. in J. Carbohydrate Chemistry, 10 (4), 549-560 (1991), which is incorporated herein by reference. That is, removal of the SE group in Compound (50) with TFA and subsequent treatment of free hydroxyl group with trichloroacetonitrile in the presence of DBU gives the α-trichloroacetimidate. The imidate is condensed with the azido sphingosin derivative derived from xylose in the presence of BF₃·OEt₂ to afford Compound (51). Selective reduction of the aside group in Compound (51) with hydrogen sulfide gives the free amino group, into which stearic acid is introduced using the carbodiimide method often employed in the synthesis of peptide to form a ceramide moiety. Finally, removal of all the protective groups for hydroxyl group with sodium methoxide yields the desired sialyl SSEA-1 ganglioside.

### INDUSTRIAL APPLICABILITY

The disaccharide derivatives of formulas (I) and (II) can be used as intermediates for the synthesis of sialyl SSEA-1 ganglioside and other lacto- and neolacto-series gangliosides which are useful as tumor markers in a serum diagnosis of cancers (Kannagi et al., Byori to Rinsho (Pathology and Clinic), Vol. 8, 6. "Glycolipids, Saccharide Chain Antigen", pp. 85-99, Bunkodo Publisher, which is incorporated herein by reference).

The invention is further illustrated by the following examples, in which the numbers of compounds are identical with those indicated in respective reaction schemes.

### EXAMPLE 1

### Synthesis of Compound (6), methyl O-(2,6-di-O-benzoyl-3,4-O-isopropylidene-β-D-galactopyranosyl)-(1→4)-3,6-di-O-benzyl-2-deoxy-2-phthalimido-1-thio-β-D-glucopyranoside

### 1-1. Synthesis of Compound (2), 6-di-O-benzoyl-3,4-O-isopropylidene-α-D-galactopyranosylbromide

Compound (1), methyl 2,6-di-O-benzoyl-3,4-O-isopropylidene-1-thio-β-D-galactopyranoside (3.0 g) was dissolved in methylene chloride (30 ml) and cooled to -20°C. To a cooled solution was added bromine (370 µl) and stirred for 20 minutes, tetraethylammonium bromide (686 mg) was added and a mixture was stirred for further 2 hrs. A reaction mixture was treated with an aqueous sodium thiosulfate solution and then washed with water. The resultant methylene chloride phase was dried over anhydrous sodium sulfate and methylene chloride was distilled away to afford 2.5 g of Compound (2), yield 76.8%.

### 1-2. Synthesis of Compound (4), methyl 3-O-benzyl-4,6-O-benzylidene-2-deoxy-2-phthalimido-1-thio-β-D-glucopyranoside

Compound (3), methyl 4,6-O-benzylidene-2-deoxy-2-phthalimido-1-thio-β-D-glucopyranoside (2.16 g) was dissolved in DMF (10 ml), to which sodium hydride (222 mg) was added and a mixture was stirred at 0°C for one hour. To the mixture was added benzyl bromide (0.72 ml) and the stirring was continued for 16 hrs. Excess methanol was added to cease the reaction. The mixture obtained by concentration was subjected to silica gel column chromatography with 5/1 hexane/ethyl acetate to afford 2.15 g of Compound (4), yield 82.2%.

### 1-3. Synthesis of Compound (5), methyl 3,6-di-O-benzyl-2-deoxy-2-phthalimido-1-thio-β-D-glucopyranoside

To a solution of Compound (4) (2.14 g) in THF (25 ml) was added sodium cyanoborohydride (3.89 g) and then hydrogen chloride/ether was added dropwise until foaming ceased. A reaction solution was neutralized with sodium hydrogencarbonate, an insoluble matter was filtered off and the solvent was distilled off. The residue was dissolved in methylene chloride, washed with water several times and dried over anhydrous sodium sulfate. The mixture obtained by concentration was subjected to silica gel column chromatography with 4/1 hexane/ethyl acetate to afford 1.82 g of Compound (5), yield 84.7%.

### 1-4. Synthesis of Compound (6)

To a solution of Compound (2) (2.27 g) and Compound (5) (1.6 g) in methylene chloride (30 ml) was added powdered molecular sieves 4A (8 g) and a mixture was stirred at room temperature for 5 hrs. A solution was cooled to -30°C, to which silver trifluoromethane sulfonate (1.58 g) was added, and the mixture was reacted for 17 hrs. A reaction solution was neutralized with sodium hydrogencarbonate, an insoluble material was filtered off and the solvent was distilled off. The residue was dissolved in methylene chloride, washed with water several times and dried over anhydrous sodium sulfate. The mixture obtained by concentration was subjected to silica gel column chromatography with 4/1 hexane/ethyl acetate to afford 1.84 g of Compound (5), yield 64.3%.

### Property of Compound (6)

C₅₂H₅₁O₁₃NSNw.=930.04
IRνₘₐₓcm⁻¹ ; 1775, 1715(imide), 1715, 1266(ester ), 874(CMe₂), 753 , 714(Ph)
¹H-NMR(300MHz,CDCl₃)
δppm ; 1.34, 1.59(each s,6H,C(CH₃)₂), 2.05(s,3H,SCH₃), 4.64(d,1H,J₁',₂' =8.5Hz, H-1' ), 5.01(d, 1H, J₁,₂=10.1Hz, H-1), 5.29(dd, 1H, J₂' ,₃'=6.9Hz, H-2 '), 4.52, 4.67, 4.73, 4.92 ( each d,4H,J_{gem}=12.4Hz,2PhCH₂O-), 6.75-8.15 (m, 24H, aromatic)
[α] _{D} ; +63.3° (C=1.2, CHCl₃)

### EXAMPLE 2

### 2-1. Synthesis of Compound (34), methyl O-(2,3,4-tri-O-benzyl-α-L-fucopyranosyl)-(1→3)-4,6-O-benzylidene-2-deoxy-2-phthalimido-1-thio-β-D-glucopyranoside

To a solution of Compound (33), methyl-2,3,4-tri-O-benzyl-1-thio-β-L-fucopyranoside (2.1 g) and Compound (3) (1.4 g) in benzene (35 ml) was added powdered molecular sieves 4A (10 g) and a mixture was stirred at room temperature for 5 hrs. A solution was cooled to 5°C, DMTST (0.78 g) was added and the stirring was continued for 2 hrs. Methanol and triethylamine were added to cease the reaction and an insoluble matter was filtered off. To the precipitate was added methylene chloride, and the solution was washed with successive, 1N hydrochloric acid, water, an aqueous sodium hydrogencarbonate solution and water, and dried over anhydrous sodium sulfate. The mixture obtained by distilling off the solvent was subjected to silica gel column chromatography with 3/1 hexane/ethyl acetate to give 2.42 g of Compound (34), yield 87.6%.

### 2-2. Synthesis of Compound (35), methyl O-(2,3,4-tri-O-benzyl-α-L-fucopyranosyl)-(1→3)-6-O-benzyl-2-deoxy-2-phthalimido-1-thio-β-D-glucopyranoside

To a solution of Compound (34) (1.07 g) in THF (14 ml) was added sodium cyanoborohydride (1.2 g) and then hydrogen chloride/ether was added dropwise until foaming ceased. A reaction solution was neutralized with sodium hydrogencarbonate, an insoluble matter was filtered off and the solvent was distilled off. The residue was dissolved in methylene chloride, washed with water several times and dried over anhydrous sodium sulfate. The mixture obtained by concentration was subjected to silica gel column chromatography with 7/2 hexane/ethyl acetate to afford 0.93 g of Compound (35), yield 87.1%.

### 2-3. Synthesis of Compound (36), methyl O-(2,3,4-tri-O-benzyl-α-L-fucopyranosyl)-(1→3)-6-O-benzyl-2-deoxy-4-O-methoxybenzyl-2-phthalimido-1-thio-β-D-glucopyranoside

Compound (35) (616 mg) was dissolved in DMF (5 ml), to which sodium hydride (32 mg) was added and a mixture was stirred at 0°C for one hour. To the mixture was added methoxybenzyl chloride (0.12 ml) and the stirring was continued for 16 hrs. Excess methanol was added to cease the reaction. The mixture obtained by concentration was subjected to silica gel column chromatography with 4/1 hexane/ethyl acetate to afford 597 mg of Compound (36), yield 84.9%.

### Property of Compound (36)

C₅₇H₅₉O₁₁NS Mw.=966.16
IRνₘₐₓcm⁻¹ ; 1774, 1712(imide, ester), 739, 721(Ph) ¹H-NMR(300MHz, CDCl₃)
δppm ; 1.02(d,3H,J₅' ,₆' =6.5Hz, H-6'), 2.17(s,3H,SCH₃), 3.76(s,3H,OCH ₃), 5.00(d,1H,J₁' ,2' =2.7Hz, H-1'), 5.31(d,1H,J₁,₂=10.4Hz, H-1), 6.76-7. 78(m, 28H, aromatic)
[α] _{D ;} +13.5° (C=0.85, CHCl₃)

### EXAMPLE 3

This example illustrates the construction of a saccharide chain moiety using Compounds (6) and (36) which were respectively synthesized in Examples 1 and 2, in accordance with the reaction processes shown in Reaction Schemes 3 and 4. This reaction was carried out in a similar manner as mentioned in Kameyama et al. in J. Carbohydrate Chemistry, 10 (4), 549-560 (1991), which is incorporated herein by reference. The physical properties of the intermediate compounds (44), (45), (46) and (47) as well as final compound (50) are listed below.

### 3-1. Property of Compound (44), 2-(trimethylsilyl)ethyl O-(2,6-di-O-benzoyl-3,4-O-isopropylidene-β-D-galactopyranosyl)-(1→4)-O-(3,6-di-O-benzyl-2-deoxy-2-phthalimido-β-D-glucopyranosyl)-(1→3)-O-(2,4,6-tri-O-benzyl-galactopyranosyl)-(1→4)-2,3,6-tri-O-benzyl-β-D-glucopyranoside

C₁₁₀H₁₁₇O₂₄NSi Mw.=1865.22
IRνₘₐₓcm⁻¹ ; 1776, 1718(imide), 1718, 1265(ester), 862, 838 (TMS), 738, 715(Ph)
¹H-NMR(300MHz, CDCl₃)
δppm ; 0.98(m, 2H, Me₃SiC H₂CH₂O), 1.36, 1.63(2s, 6H, C(CH₃)₂), 5.29(d, 1H, J₁,₂=8.3Hz, H-1 GlcN), 6.75-8.14(m, 54H, aromatic)
[α] _{D} ; +13.5° (C=0.77, (CHCl₃)

### 3-2. Property of Compound (45), 2-(trimethylsilyl)ethyl O-(2,6-di-O-benzoyl-β-D-galactopyranosyl)-(1→4)-O-(3,6-di-O-benzyl-2-deoxy-2-phthalimido-β-D-glucopyranosyl)-(1→3)-O-(2,4,6-tri-O-benzyl-β-D-galactopyranosyl)-(1→ 4)-2,3,6-O-benzyl-β-D-glucopyranoside

C₁₀₇H₁₁₃O₂₄NSi Nw.=1825.15
RIνₘₐₓcm⁻¹ ; 3464(OH), 1777, 1717(imide), 1717, 1269( ester ), 860 , 839(TMS), 739, 717, 700(Ph)
¹H-NMR(300MHz, CDCl₃)
δppm ; 0.98(m, 2H, Me₃SiC H₂CH₂O), 5.32(d, 1H, J₁,₂=8.2Hz, H-1 GlcN), 6.78-8.11(m, 54H, aromatic)
[α] _{D} ; +6.0° (C=1.07, CHCl₃)

### 3-3. Property of Compound (46), 2-(trimethylsilyl)ethyl O-(2,3,4-tri-O-benzyl-α-L-fucopyranosyl)-(1→3)-O-(6-O-benzyl-2-deoxy-4-O-methoxybenzyl-2-phthalimido-β-D-glucopyranosyl)-(1→3)-O-(2,6-di-O-benzoyl-β-D-galactopyranosyl)-(1→4)-O-(3,6-di-O-benzyl-2-deoxy-2-phthalimido-β-D-glucopyranosyl)-(1→3)-O-(2,4,6-tri-O-benzyl-β-D-galactopyranosyl)-(1→4)-2,3,6-O-benzyl-β-D-glucopyranoside

C₁₆₃H₁₆₈O₃₅N₂Si Mw.=2743.20
IRνₘₐₓcm⁻¹ ; 3544(OH), 1776, 1715(imide, ester), 868(TMS), 739, 701(Ph)
¹H-NMR(300MHz, CDCl₃)
δppm ; 0.98(m, 2H, Me₃SiC H₂CH₂O), 1.04(d, 3H, J₅,₆=6.5Hz, H-6 Fuc), 3.79(s , 3H, OCH₃), 5.18(d, 1H, J₁,₂=8.1Hz, H-1 GlcN), 5.41(dd, 1H, J₁,₂=8.4Hz, J₂,₃=9. 3Hz, H-2 Gal), 5.48(d, 1H, J₁,₂=8.2Hz, H-1 GlcN), 6.73-8.12(m, 82H, aromatic)
[α] _{D} ; +9.9° (C=0.63, CHCl₃)

### 3-4, Property of Compound (47), 2-(trimethylsilyl)ethyl O-(2,3,4-tri-O-benzyl-α-L-fucopyranosyl)-(1→3)-O-(2-acetamido-6-O-benzyl-2-deoxy-β-D-glucopyranosyl)-(1→ 3)-O-(2,4,6-tri-O-acetyl-β-D-galactopyranosyl)-(1→4)-O-(2-O-acetamido-3,6-di-O-benzyl-2-deoxy-β-D-glucopyranosyl)-(1→3)-O-(2,4,6-tri-O-benzyl-β-D-galactopyranosyl)-(1→4)-2,3,6-O-benzyl-β-D-glucopyranoside

C₁₃₅H₁₅₈O₃₃N₂Si Mw.=2364.82
IRνₘₐₓcm⁻¹ ; 3390(OH, NH), 1750, 1225(ester ), 1669, 1533(amide), 859, 838(TMS), 738, 699(Ph)
¹H-NMR(300MHz, CDCl₃)
δppm ; 1.00(m, 2H, Me₃SiC H₂CH₂O), 1.14(d, 3H, J₅,₆=6.5Hz, H-6 Fuc), 1.45, 1.58(2s, 6H, 2AcN), 1.89, 1.98, 2.06(3s, 9H, 3AcO), 5.37(d, 1H, J₃,₄=3.4Hz, H-4 Gal), 7.07-7.43(m, 60H, aromatic)
[α] _{D} ; -15.6° (C=0.68, CHCl₃)

### 3-5. Property of Compound (49), 2(trimethylsilyl)ethyl O-(methyl 5-acetamido-4,7,8,9-tetra-O-acetyl-3,5-dideoxy-D-glycero-α-D-galacto-2-nonulopyranosylonate)-(2→3)-O-(2,4,6-tri-O-benzyl-α-L-fucopyranosyl)-(1→3)-O-(2-acetamido-6-O-benzyl-2-deoxy-β-D-glucopyranosyl)-(1→3)-(2,4,6-tri-O-acetyl-β-D-galactopyranosyl)-(1→4)-O-(2-O-acetamido-3,6-di-O-benzyl-2-deoxy-β-D-glucopyranosyl)-(1→3)-O-(2,4,6-tri-O-benzyl-β-D-galactopyranosyl)-(1→4)-2,3,6-O-benzyl-β-D-glucopyranoside

C₁₈₂H₂₀₇O₅₃N₃Si Nw.=3312.72
IRνₘₐₓcm⁻¹ ; 3380(NH), 1742, 1266(ester ), 1663, 1534(amide), 860 , 834(TMS), 739, 714, 701(Ph)
¹H-NMR(300MHz, CDCl₃)
δppm ; 0.98(m, 2H, Me₃SiC H₂CH₂O), 1.03(d, 3H, J₅,₆=6.5Hz, H-6 Fuc), 1.46, 1.54, 1.59(3s, 9H, 3AcN), 1.78-2.21(7s, 21H, 7AcO), 2.43(dd, 1H, J_{gem}=12.8Hz, J ₃ₑ,₄=4.6Hz, H-3e Neu), 3.81(s, 3H, OCH₃), 5.49(dd, 1H, J₁,₂=8.4Hz, J₂,₃=9.6Hz, H-2 Gal), 7.08-8.09(m, 75H, aromatic)
[α] _{D} ; -6.0° (C=0.315, CHCl₃)

### 3-6. Property of Compound (50), 2-(trimethylsilyl)ethyl O-(methyl 5-acetamido-4,7,8,9-tetra-O-acetyl-3,5-dideoxy-D-glycero-α-D-galacto-2-nonulopyranosylonate)-(2→3)-O-(2,4,6-tri-O-acetyl-α-L-fucopyranosyl)-(1→3)-O-(2-acetamido-6-O-acetyl-2-deoxy-β-D-glucopyranosyl)-(1→3)-(2,4,6-tri-O-acetyl-β-D-galactopyranosyl)-(1→4)-O-(2-O-acetamido-3,6-di-O-acetyl-2-deoxy-β-D-glucopyranosyl)-(1→3-)O-(2,4,6-tri-O-acetyl-β-D-galactopyranosyl)-(1→4)-2,3,6-O-acetyl-β-D-glucopyranoside

C₁₂₂H₁₅₉O₆₅N₃Si Nw.=2735.67
¹H-NMR(300MHz, CDCl₃)
δppm ; 0.98(m, 2H, Me₃SiCH₂CH₂O), 1.02(d, 3H, J₅,₆=6.4Hz, H-6 Fuc), 1.44, 1 .54, 1.62(3s, 9H, 3AcN), 1.78-2.15(19s, 57H, 19AcO), 2.46(dd, 1H, J_{gem}=12.5Hz , J₃ₑ,₄=4Hz, H₃ₑ Neu), 3.80(s, 3H, MeO), 4.98(d, 1H, J₁,₂=3.0Hz, H-1 Fuc), 5. 15(d, 1H, J₁,₂=8.4Hz, H-1 Gal), 5.43(dd, 1H, J₂,₃=9.7Hz, H-2 Gal), 5.57(d, 1H, J ₃,₄=3.3Hz, H-4 Gal), 5.61(m, 1H, H-8 Neu), 7.21-8.05(m, 15H, aromatic)
[α] _{D} ; -13.7° (C=0.80, CHCl₃)
In accordance with Reaction Scheme 5, a ceramide moiety was introduced into Compound (50) to give sialyl SSEA-1 ganglioside (VI³NeuAcV³FucnLc₆-Cer) having the following physical properties.

### 3-7. Property of sialyl SSEA-1 ganglioside (VI³NeuAcV³FucnLc₆-Cer)

¹H-NMR(300MHz, 49 : 1(CD₃)₂SO-D₂O)
δppm ; 0.85(t, 6H, 2CH₃CH₂), 1.01(d, 3H, J₅,₆=6.4Hz, H-6 Fuc), 1.23(s, 52H, 2 6CH₂), 1.77, 1.81, 1.90(3s, 9H, 3AcN), 2.03(t, 2H, COCH₂CH₂), 2.74(dd, 1H, J_{ge} m=12.5Hz, J₃ₑ,₄=4.7Hz, H₃ₑ Neu), 4.15(d, 1H, J₁,₂=7.4Hz, H-1 Glc), 4.65(d, 1H, J₁,₂=7.3Hz, H-1 GlcNAc), 4.72(d, 1H, J₁,₂=7.3Hz, H-1 GlcNAc), 4.82(d, 1H, J₁,₂ =3.3Hz, H-1 Fuc), 5.38(dd, 1H, J₃,₄=7.0Hz, J₄,₅=15.2Hz, H-4 sphingosine) 5.54(dt, 1H, J₅,₆=J₅,₆=7.1Hz, H-5 sphingosine)
[α] _{D} ; -18.5° (C=0.63, 5 : 4 : 0.7 CHCl₃-CH₃OH-H₂O)

## Claims

1. A disaccharide derivative selected from the group consisting of the compounds represented by formulas (I) and (II) 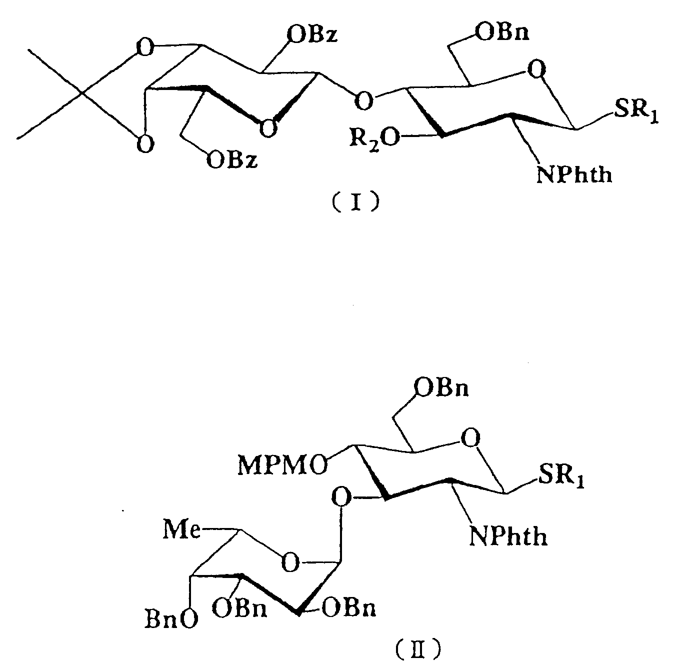 in which R₁ represents C₁-C₄ alkyl or phenyl, R₂ represents benzyl or methoxybenzyl, Bn stands for benzyl, Bz stands for benzoyl, MPM stands for methoxybenzyl, Me stands for methyl and NPhth stands for phthalimide of the formula

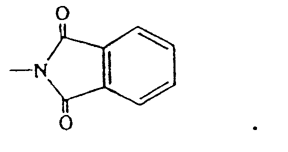

2. A disaccharide derivative of formula (I) 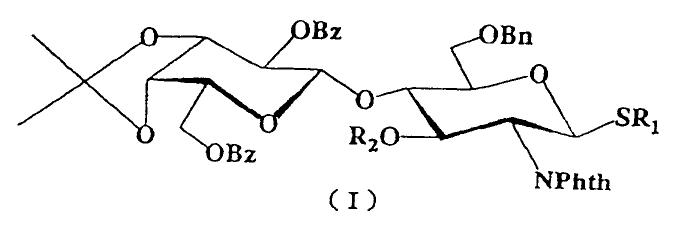 in which R₁ represents C₁-C₄ alkyl or phenyl, R₂ represents benzyl or methoxybenzyl, Bn stands for benzyl, Bz stands for benzoyl and NPhth stands for phthalimide of the formula

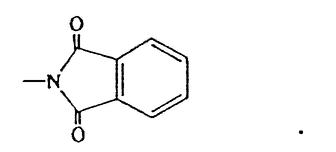

3. A disaccharide derivative of formula (II) 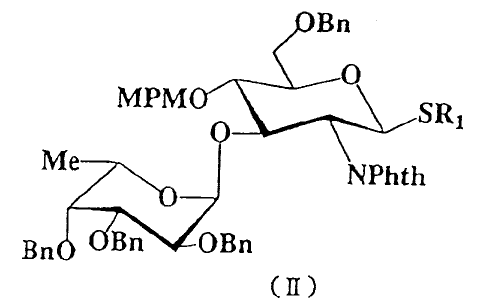 in which R₁ represents C₁-C₄ alkyl or phenyl, Bn stands for benzyl, MPM stands for methoxybenzyl, Me stands for methyl and NPhth stands for phthalimide of the formula

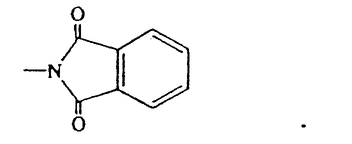

4. A process of preparing a disaccharide derivative of formula (I) 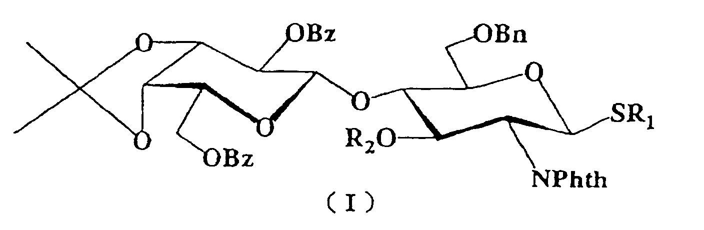 in which R₁ represents C₁-C₄ alkyl or phenyl, R₂ represents benzyl or methoxybenzyl, Bn stands for benzyl, Bz stands for benzoyl and NPhth stands for phthalimide of the formula 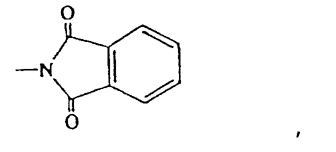 which comprises reacting a compound of formula (2) 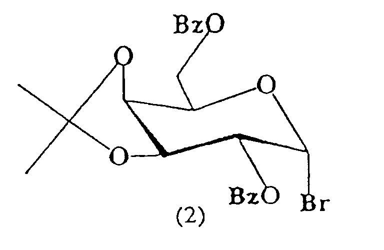 with a compound of formula (III) 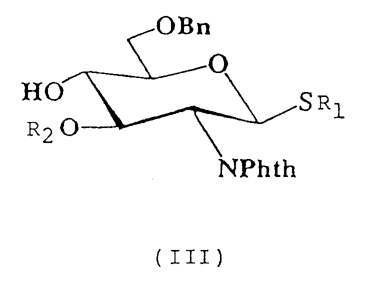 in which R₁ represents C₁-C₄ alkyl or phenyl, Bn stands for benzyl and NPhth stands for phthalimide of the fomula

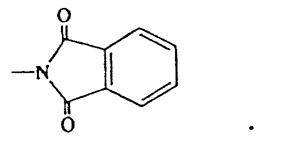

5. A process of Claim 4 wherein the compound of formula (2) is prepared by brominating the anomeric position of a compound having the following formula 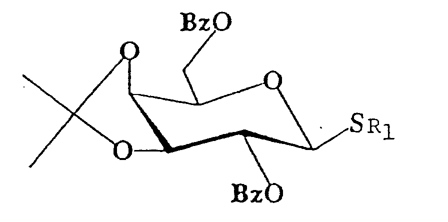 in which R₁ and Bz are as defined above.

6. A process of Claim 4 wherein the compound of formula (III) is prepared by protecting a free hydroxyl group of the following formula 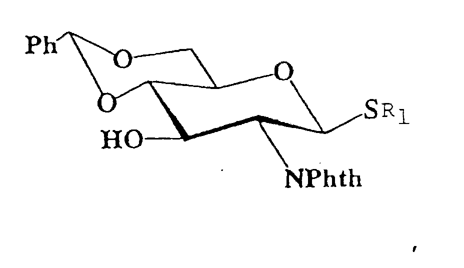 with a benzyl or substituted benzyl group to form a compound of the following formula 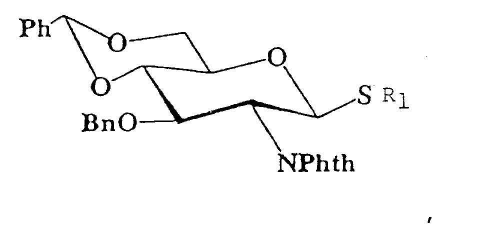 followed by reductive cleavage of the resultant compound.

7. A process of preparing a disaccharide derivative of formula (II) 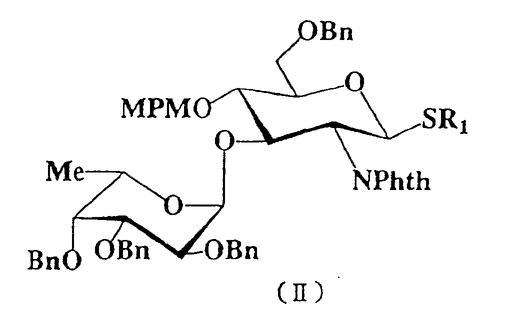 in which R₁ represents C₁-C₄ alkyl or phenyl, Bn stands for benzyl, MPM stands for methoxybenzyl, Me stands for methyl and NPhth stands for phthalimide of the formula 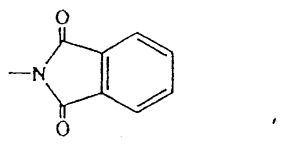 which comprises reacting a compound of the following formula 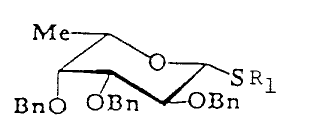 with a compound of the following formula 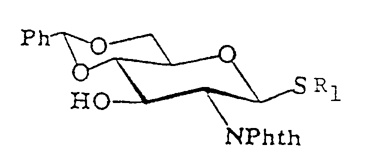 to form a compound of the following formula 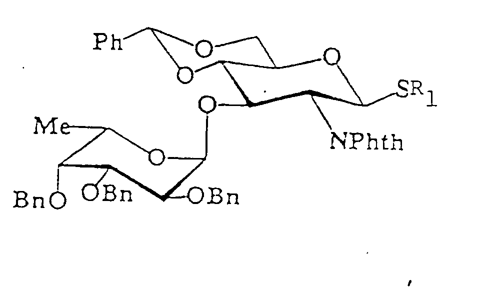 followed by reductive cleavage of the resultant compound to form a compound of the following formula 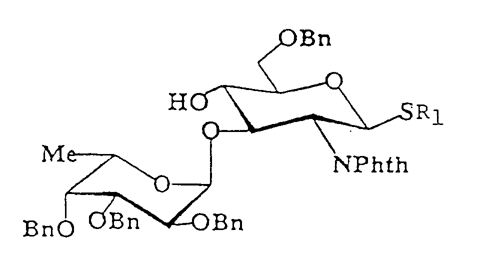 and methoxybenzylation of the resultant compound.

8. Use of the disaccharide derivatives of any of claims 1 to 3 for the preparation of gangliosides.
